(19) Europäisches Patentamt
European Patent Office
Office européen des brevets

(11) Numéro de publication: **0 094 271**
**B1**

(12) # FASCICULE DE BREVET EUROPEEN

(45) Date de publication du fascicule du brevet: **06.08.86**

(21) Numéro de dépôt: **83400749.4**

(22) Date de dépôt: **15.04.83**

(51) Int. Cl.⁴: **C 07 D 217/26**, C 07 D 215/48, C 07 D 239/72, C 07 D 495/04, C 07 D 409/04, C 07 C 103/76, A 61 K 31/165, A 61 K 31/44, A 61 K 31/47, A 61 K 31/505 // (C07D495/04, 333:00, 221:00)

(54) **Nouveaux dérivés d'arène et d'hétéroarènecarboxamides, leur procédé de préparation et médicaments les contenant.**

(30) Priorité: **27.04.82 FR 8207217**

(43) Date de publication de la demande: **16.11.83 Bulletin 83/46**

(45) Mention de la délivrance du brevet: **06.08.86 Bulletin 86/32**

(84) Etats contractants désignés: **BE CH DE FR GB IT LI LU NL SE**

(56) Documents cités:
**EP - A - 0 003 920**
**BE - A - 764 299**

**JOURNAL OF ORGANIC CHEMISTRY, vol. 32, no. 12, décembre 1967, pages 3798-3803, Washington, US R.I. FRYER et al.: "Quinazolines and 1,4-Benzodiazepines. XXXVII. Synthesis and rearrangements of a substituted 5-phenyl-1H-1,4-benzodiazepine"**
**CHEMICAL ABSTRACTS, vol. 98, no. 19, 9 mai 1983, page 36, no. 155122p, Columbus, Ohio, US S.E. FILE et al.: "The anxiogenic action of Ro 5-4864 is reversed by phenytoin"**

(73) Titulaire: **PHARMUKA LABORATOIRES, 35 Quai du Moulin de Cage, F-92231 Gennevilliers (FR)**

(72) Inventeur: **Dubroeucq, Marie-Christine, 13 Villa Malleville, F-95880 Enghien-les-Bains (FR)**
Inventeur: **Renault, Christian Louis Albert, 10 Allée Réaumur, F-95150 Taverny (FR)**
Inventeur: **Le Fur, Gérard Roger, 35, rue du Progrès, F-92350 Plessis-Robinson (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 094 271 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet de nouveaux dérivés d'arène et d'hétéroarènecarboxamides utilisables comme médicaments et leur procédé de préparation.

Les divers effets cliniques (anxiolytique, anticonvulsivant, hypnotique, myorelaxant) des benzodiazépines ont été attribués à la présence, dans le système nerveux central des mammifères, de sites de liaisons saturables, à haute affinité et stéréospécifiques. (C. Braestrup et Coll. Nature 1977, 269, 702; J. F. Tallman et Coll. Science, 1980, 207, 274).

Certaines benzodiazépines se lient aussi à des membranes de tissus périphériques comme le rein avec également une forte affinité (C. Braestrup et Coll. Proc. Natl. Acad. Sci. USA, 1977, 74, 3805). Les deux récepteurs de benzodiazépines présents dans ces tissus diffèrent de ceux marqués par le [$^3$H] diazépam et le [$^3$H] flunitrazépam dans le cerveau; par exemple le clonazépam qui a une très forte affinité pour les sites de liaison du [$^3$H] diazépam du cerveau est pratiquement inactif à l'égard des sites de liasison du [$^3$H] diazépam dans le rein. A l'inverse un dérivé chloré du diazépam le Ro-5-4864 est très actif au niveau périphérique mais inactif au niveau central. Ainsi, il est possible de distinguer au moins 2 types de récepteurs de benzodiazépines, l'un de type «cérébral» dont le critère pharmacologique sera un classement par affinité décroissante dans l'ordre suivant: clonazépam > diazépam > Ro-5-4864 et l'autre de type «périphérique» dont le critère pharmacologique sera un classement par affinité décroissante dans l'ordre suivant: Ro-5-4864 > diazépam > clonazépam.

Ces récepteurs de type «périphérique» sont présents dans de nombreux organes: le cœur, le rein, le poumon, les plaquettes sanguines et également le cerveau (où sont donc présents les 2 types de récepteurs) L.P. Davies et Coll. Eur. J. Pharmacol. 1981, 73, 209; J.K.T Wang et Coll. Life Sciences 1980, 27, 1881; J.W. Regan et Coll. Life Sciences 1981, 28, 991; H. Schoemaker, Eur. J. Pharmacol., 1981, 71, 1973). Enfin, il a été montré la présence de ces récepteurs de type «périphérique» dans les thymocytes (J.K.T. Wang et Coll. Pharmacologist, 1981, 23, 160) et que ces récepteurs régulent la prolifération des cellules de thymones (J.K.T. Wang et Coll. Fed. Proc. 1982, 41, 1328).

Les thiéno [3,2-c] et thiéno [2,3-c] pyridines du brevet EP-A-3920 montrent des activités sédative, anti-convulsivante et anti-inflammatoire.

Les composés selon l'invention qui, bien que de structures différentes de celles des benzodiazépines, se lient spécifiquement aux récepteurs des benzodiazépines de type «périphérique».

Ces composés sont représentés par la formule générale:

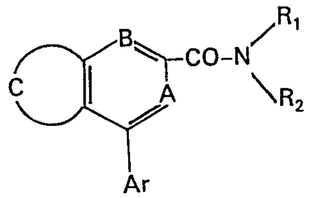

(I)

dans laquelle $R_1$ et $R_2$ représentent indépendamment un groupe alkyle comportant de 1 à 6 atomes de carbone, linéaire ou ramifié, un groupe cycloalkyle comportant de 3 à 7 atomes de carbone, un groupe phénylalkyle ou cycloalkylalkyle, dont la partie alkyle comporte de 1 à 3 atomes de carbone. $R_1$ et $R_2$ peuvent représenter également un groupe alcényle ou alcynyle comportant de 3 à 6 atomes de carbone, à condition que la double ou la triple liaison ne soit pas situés en position 1-2 par rapport à l'atome d'azote. $R_1$ et $R_2$ peuvent représenter encore un groupe de formule $-R_3-Z-R_4$ dans laquelle $R_3$ représente un groupe alkylène linéaire ou ramifié de 2 à 6 atomes de carbone, à condition qu'au moins 2 atomes de carbone séparent l'atome d'azote du groupe Z; $R_4$ représente un groupe alkyle comportant de 1 à 4 atomes de carbone et Z l'atome d'oxygène, de soufre ou le groupe $>N-R_5$, $R_5$ représentant l'atome d'hydrogène ou un groupe alkyle de 1 à 3 atomes de carbone.

En outre $R_1$ et $R_2$ peuvent former avec l'atome d'azote auquel ils sont rattachés un hétérocycle qui peut comporter un second hétéroatome: azote ou oxygène; c'est-à-dire que

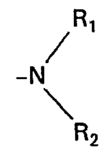

peut représenter un cycle pyrrolidine, pipéridine, morpholine ou pipérazine éventuellement substitué à l'azote par un groupe alkyle comportant de 1 à 3 atomes de carbone.

Ar représente un groupe phényle, pyridyle ou thiènyle ou un groupe phényle substitué par un ou deux substituants pris parmi les atomes d'halogène (chlore, fluor, brome), les groupes alkyle, alcoxy et alkylthio ayant 1 à 4 atomes de carbone, le groupe trifluorométhyle et le groupe nitro.

Les groupes A et B représentent indépendamment N ou CH-

Le groupe C< représente l'enchaînement

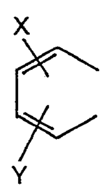

ou, lorsque A représente N et B représente CH, l'un des enchaînements suivants:

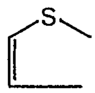

,

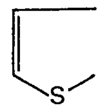

Autrement dit les produits de formule (I) de la présente invention répondent à l'une des 3 formules ci-dessous:

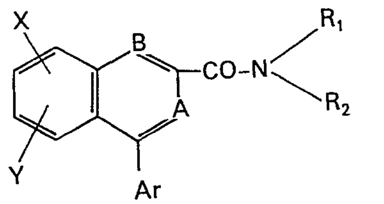

(II)

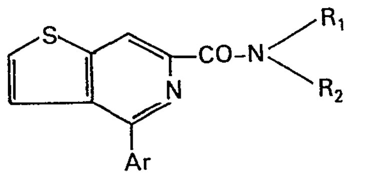

(III)

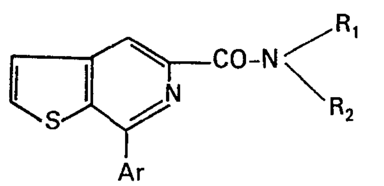

(IV)

Dans les formules II, III et IV, $R_1$, $R_2$, Ar, A et B ont les significations exposées ci-dessus; X et Y représentent indépendamment un atome d'hydrogène, d'halogène (fluor, chlore, brome), un groupe alkyle ou alcoxy comportant de 1 à 3 atomes de carbone, le groupe nitro ou trifluorométhyle.

Lorsque le groupe

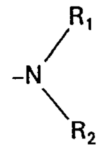

comporte un atome de carbone asymétrique les produits de formule (I) sont dédoublables en inverses optiques. Dans ce cas chaque énantiomère et le composé racémique font partie de l'invention.

Les produits de formule générale (I) peuvent être préparés à partir des acides de formule (V) et des amines

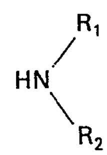

par les méthodes connues permettant de transformer un acide carboxylique en carboxamide, en passant par un dérivé réactif (VI) de l'acide carboxylique, selon le schéma suivant:

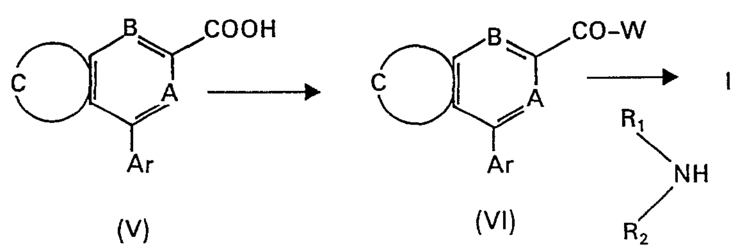

(V) (VI)

Les dérivés réactifs de formule (VI) les plus usités sont les esters (W représente un groupe alcoxy de bas poids moléculaire), les chlorures d'acides (W représente l'atome de chlore) et les anhydrides (W représente un groupe alcoxycarbonyloxy de bas poids moléculaire), (cf. C.A. Buehler et D.E. Pearson, Survey of Organic Synthesis. Wiley Interscience, 1970, page 894).

Les esters de formule (VI) peuvent être préparés par chauffage des acides de formule (V) au sein d'un alcool à bas poids moléculaire tel que le méthanol ou l'éthanol, au reflux et en présence d'un acide minéral tel que l'acide sulfurique ou l'acide chlorhydrique. Les esters ainsi obtenus sont ensuite traités par au moins un équivalent d'amine

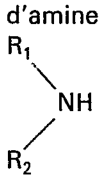

en présence d'un agent de métallation tel que le butyllithium, au sein d'un solvant inerte tel que l'éther éthylique ou le tétrahydrofuranne, à une température comprise entre −10°C et + 30°C.

Les chlorures d'acide de formule (VI) peuvent être préparés par action sur les acides correspondants de formule (V) d'un agent de chloruration tel que le chlorure de thionyle en l'absence de solvant ou au sein d'un solvant inerte tel que le chloroforme et au reflux. On traite ensuite le chlorure d'acide brut ainsi obtenu par l'amine

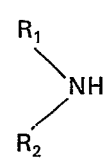

au sein d'un solvant inerte tel que le toluène ou le chloroforme et à température ambiante. On opère avantageusement en présence d'un excès d'au moins un équivalent d'amine qui joue le rôle de

base neutralisant l'acide chlorhydrique formé. On peut aussi opérer au sein de la pyridine qui sert à la fois de base et de solvant.

Les anhydrides mixtes de formule (VI) peuvent être préparés en traitant les acides correspondants de formule (V) par un chloroformiate d'alkyle C1-COO-R' (R' représentant un groupe alkyle de bas poids moléculaire tel que méthyle ou éthyle), en présence d'une amine tertiaire telle que la triéthylamine; le produit obtenu est ensuite traité par l'amine

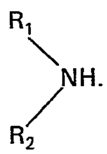

Il est avantageux d'effectuer les deux réactions dans le même appareillage, sans isoler l'anhydride mixte. Celui-ci est préparé à une température comprise entre −5°C et +25°C, au sein d'un solvant inerte tel que le chloroforme ou le chlorure de méthylène et on ajoute ensuite l'amine

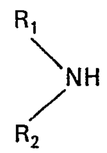

pure ou en solution dans un solvant inerte tel que le benzène, le toluène, le chloroforme ou le chlorure de méthylène, à la même température.

Les acides carboxyliques de formule générale (V), suivant les significations de A, B et C appartiennent à l'une des familles définies par les formules ci-dessous:

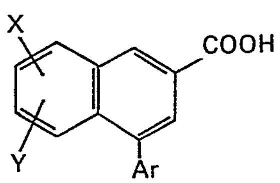

(VII)

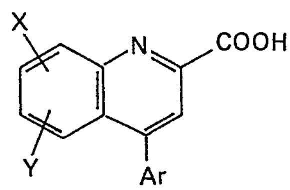

(VIII)

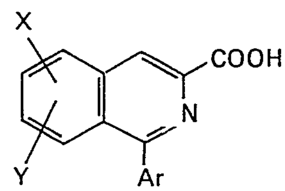

(IX)

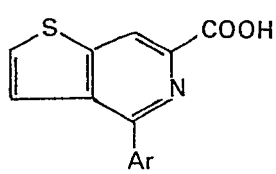

(X)

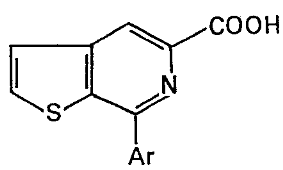

(XI)

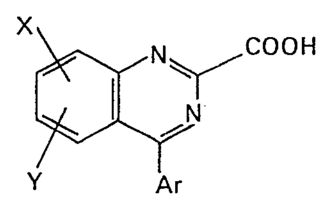

(XII)

Certains composés de formule (VII) sont connus; en particulier l'acide phényl-4 naphtalènecarboxylique-2. Les composés de formule (VII) peuvent être préparés en appliquant aux précurseurs appropriés les réactions utilisées pour la préparation des dérivés connus (R. Filler et Coll. J. Org. Chem. 1962, 27, 4440).

De même, les acides carboxyliques de formule (VIII) peuvent être préparés par analogie avec le procédé décrit pour la préparation des produits de formule (VIII) connus (K. Takahashi et Coll. J. Heterocycl. Chem. 1977, 14, 881).

Parmi les composés de formule (IX), quelques uns sont connus, en particulier l'acide phényl-1 isoquinoléinecarboxylique-3. (R. Filler et Coll. J. Org. Chem. 1962, 27, 2403; D.A. WALSH et Coll. J. Med. Chem. 1978, 21, 582). Les procédés décrits dans ces publications peuvent être utilisés de façon analogue pour la préparation des produits de formule (IX), en partant des précurseurs appropriés.

Les composés de formule (X) et (XI) sont des produits nouveaux. Pour leur préparation il est avantageux d'utiliser un procédé analogue à celui décrit par R. Filler et Coll. (J. Org. Chem. 1962, 27, 2403), selon le schéma réactionel ci-dessous:

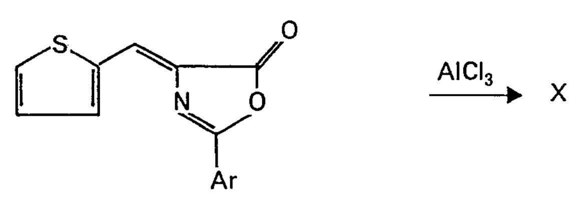


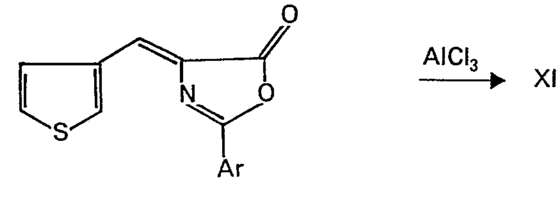


Pour effectuer ces réactions, les oxazolones de départ sont traitées par 3 équivalents de chlorure d'aluminium au sein du tétrachloro-1, 1,2,2 éthane, à une température comprise entre 20°C et la température d'ébullition du solvant.

Quelques composés de formule (XII) sont connus (notamment les produits pour lesquels X représente l'atome d'hydrogène, Y l'atome de chlore en position 6 et Ar le groupe phényle ou chloro-2 phényle) et ont été obtenus à partir de benzodiazépines (STERNBACH et Coll. J. Org. Chem. 1964, 29, 332). Néanmoins, il est plus avantageux de préparer ces composés ainsi que tous les composés de formule (XII) à partir des aroyl-2 anilines de formule (XIII), selon le schéma réactionnel suivant:

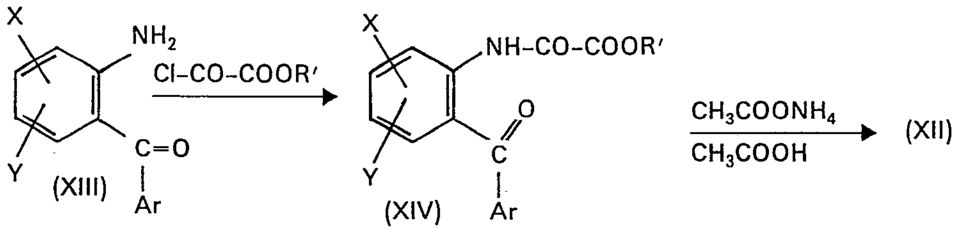


(XIII) (XIV) (XII)

Les aroyl-2 anilines de formule (XIII) sont traités par au moins un équivalent d'un chlorure d'alcoyloxalyle Cl-CO-COOR' (R' représentant un groupe alkyle de bas poids moléculaire tel que méthyle ou éthyle), au sein de la pyridine, à une température comprise entre 0 et 25°C. Le composé de formule (XIV) ainsi préparé est ensuite traité par un grand excès d'acétate d'ammonium au sein de l'acide acétique au reflux.

Les mélanges réactionnels obtenus par les divers procédés décrits précédemment sont traités suivant des méthodes classiques, physiques (évaporation, extraction à l'aide d'un solvant, distillation, cristallisation, chromatographie, etc....) ou chimiques le cas échéant (formation de sel et régénération de la base ou de l'acide, etc....) afin d'isoler les composés de formule (I) à l'état pur.

Les exemples suivants illustrent l'invention sans la limiter.

Exemple 1

N,N-diéthyl phényl-4 naphtalènecarboxamide-2.

On porte 4 heures à 80°C, 4 g d'acide phényl-4 naphtalènecarboxylique-2 dans 16 ml de chlorure de thionyle puis on concentre à sec sous pression réduite. On reprend le résidu dans 20 ml de pyridine puis ajoute 3,8 ml de diéthylamine. On agite 2 heures à la température ambiante, verse le mélange réactionnel dans 200 ml d'eau et extrait l'insoluble par 3 fois 80 ml d'éther. On lave la phase organique par 3 fois 50 ml d'eau, 3 fois 50 ml d'acide chlorhydrique 0,1 N et 1 fois 80 ml d'eau. On la sèche sur du sulfate de magnésium et l'évapore à sec sous pression réduite. Après recristallisation du solide résiduel dans l'éther éthylique on obtient 2 g de N,N-diéthyl phényl-4 naphtalènecarboxamide-2 fondant à 130°C.

L'acide phényl-4 naphtalènecarboxylique-2 peut être obtenu selon R. Filler et Coll. J. Org. Chem., 1962, 27, 4440.

Exemple 2

N-méthyl N-(méthyl-1 propyl) phényl-4 naphtalènecarboxamide-2.

On opère comme dans l'exemple 1 à partir de 4,3 g d'acide phényl-4 naphtalènecarboxylique-2, 20 ml de chlorure de thionyle puis 1,5 g de N-méthyl butanamine-2 dans 20 ml de pyridine. On obtient 3 g de N-méthyl N-(méthyl-1 propyl) phényl-4 naphtalènecarboxamide-2 fondant à 108°C.

Exemple 3

N,N-diéthyl phényl-4 quinoléinecarboxamide-2.

On procède comme à l'exemple 1 en partant de 2 g d'acide phényl-4 quinoléinecarboxylique-2 de 6 ml de chlorure de thionyle et de 8,3 ml de diéthylamine. On isole après traitement 2,3 g de N,N-diéthyl phényl-4 quinoléinecarboxamide-2 sous forme d'une huile orangée. Après cristallisation dans un mélange hexane-éther 50/50, le produit présente un point de fusion inférieur à 50°C.

L'acide phényl-4 quinoléinecarboxylique-2 peut être préparé selon: Takahashi et Mitsuhashi, J. Het. Chem. (15) p. 881 (1977).

Exemple 4

N,N-diméthyl phényl-1 isoquinoléinecarboxamide-2.

A 1,1 g de diméthylamine dans 20 ml de tétrahydrofuranne anhydre on ajoute, à −10°C et sous azote, 10,5 ml d'une solution 1,6 M de butyllithium dans l'hexane. Après 45 mn d'agitation, on ajoute 2,2 g de phényl-1 isoquinoléinecarboxylate-3 d'éthyle et on agite 4 h à la température ambiante. On ajoute 50 ml d'eau et extrait l'insoluble avec 3 fois 100 ml d'acétate d'éthyle. On sèche la phase organique sur du sulfate de magnésium et l'évapore à sec sous pression réduite. On recristallise le résidu dans de l'éther isopropylique, on obtient 1,5 g de N,N-diméthyl phényl-1 isoquinoléinecarboxamide-3 fondant à 157°C.

Le phényl-1 isoquinoléinecarboxylate-3 d'éthyle peut être préparé par action de l'éthanol sur l'acide phényl-1 isoquinoléinecarboxylique-3 en présence d'acide sulfurique concentré. Il a un point de fusion de 114–116°C.

L'acide phényl-1 isoquinoléinecarboxylique-3 peut être préparé selon R. Filler et Coll. J. Org. Chem., 1962, 27, 2403.

Exemple 5

N,N-diéthyl phényl-1 isoquinoléinecarboxamide-3.

On opère comme dans l'exemple 4 à partir de

3,9 g de phényl-1 isoquinoléinecarboxylate-3 d'éthyle, de 3,4 g de diéthylamine et de 19 ml d'une solution 1,6 M dans l'hexane de butyllithium. On obtient 1,75 g de N,N-diéthyl phényl-1 isoquinoléinecarboxamide-3 fondant à 72°C.

Exemple 6

N,N-diéthyl (chloro-2 phényl)-1 isoquinoléinecarboxamide-3.

On opère dans l'exemple 4 à partir de 3,4 g de (chloro-2 phényl)-1 isoquinoléinecarboxylate-3 d'éthyle, de 2,4 g de diéthylamine et de 14 ml d'une solution 1,6 M de butyllithium dans l'hexane. Après chromatographie sur du gel de silice avec un mélange cyclohexane-acétate d'éthyle 1:1) comme éluant et recristallisation dans l'éther isopropylique, on obtient 0,85 g de N,N-diéthyl (chloro-2 phényl)-1 isoquinoléinecarboxamide-3 fondant à 112°C.

Le (chloro-2 phényl)-1 isoquinoléinecarboxylate-3 d'éthyle peut être préparé par action de l'éthanol sur l'acide (chloro-2 phényl)-1 isoquinoléinecarboxylique-3, en présence d'acide sulfurique concentré. Il a un point de fusion de 126°C.

L'acide (chloro-2 phényl)-1 isoquinoléinecarboxylique-3 peut être obtenu par action, à la température ambiante, du chlorure d'aluminium (0,165 mole) sur la (chloro-2 phényl)-2 phénylméthylène-4 4H-oxazolone-5 (0,055 mole) dans 250 ml de tétrachloroéthane. Il a un point de fusion de 195–196°C.

Exemple 7

[(Phényl-1 isoquinolyl)-3 carbonyl]-1 pipéridine.

A 3,74 g d'acide phényl-1 isoquinoléinecarboxylique-3 dans 150 ml de chloroforme on ajoute 1,73 g de triéthylamine. On refroidit à 10°C et ajoute 1,86 g de chloroformiate d'éthyle. On agite 40 mn à la température ambiante, introduit une solution de 1,63 g de pipéridine dans 10 ml de toluène et agite 4 h à la température ambiante. On évapore le milieu réactionnel sous pression réduite, reprend le résidu dans de l'acétate d'éthyle, lave la phase organique avec une solution aqueuse saturée de carbonate de sodium, la sèche sur du sulfate de magnésium et l'évapore à sec sous pression réduite. On chromatographie le résidu sur du gel de silice avec un mélange cyclohexaneacétate d'éthyle (1:1) comme éluant et recristallise le produit dans de l'éther isopropylique. On obtient 2,4 g de [(phényl-1 isoquinolyl)-3 carbonyl]-1 pipéridine fondant à 179°C.

Exemple 8

[(Phényl-1 isoquinolyl)-3 carbonyl]-4 morpholine.

On opère comme dans l'exemple 7 à partir de 3,74 g d'acide phényl-1 isoquinoléinecarboxylique-3, de 1,73 g de triéthylamine, de 1,86 g de chloroformiate d'éthyle, et de 1,67 g de morpholine dans 150 ml de chloroforme.

On obtient 3 g de [(phényl-1 isoquinolyl)-3 carbonyl]-4 morpholine fondant à 178°C.

Exemple 9

Méthyl-4 [phényl-1 isoquinolyl)-3 carbonyl]- pipérazine.

On opère comme dans l'exemple 7 à partir de 3,74 g d'acide phényl-1 isoquinoléinecarboxylique-3, de 1,73 g de triéthylamine, de 1,86 g de chloroformiate d'éthyle et de 1,92 g de N-méthylpipérazine dans 150 ml de chloroforme. On obtient 1,7 g de méthyl-4 [(phényl-1 isoquinolyl)-3 carbonyl]-1 pipérazine fondant à 127°C.

Exemple 10

N-méthyl N-(méthyl-1 propyl) phényl-1 isoquinoléinecarboxamide-3.

On opère comme à l'exemple 7 partir de 2,96 g d'acide phényl-1 isoquinoléinecarboxylique-3, de 1,33 g de triéthylamine, de 1,49 g de chloroformiate d'éthyle et de 1,34 g de N-méthyl butanamine-2 dans 120 ml de chloroforme. On obtient 2,4 g de N-méthyl N-(méthyl-1 propyl) phényl-1 isoquinoléinecarboxamide-3 fondant à 95–96°C.

Exemple 11

N,N-diéthyl (chloro-3 phényl)-1 isoquinoléinecarboxamide-3.

On opère comme dans l'exemple 7 à partir de 1,7 g d'acide (chloro-3 phényl)-1 isoquinoléinecarboxylique-3, de 0,69 g de triéthylamine, de 0,74 g de chloroformiate d'éthyle et de 0,56 g de diéthylamine dans 60 ml de chloroforme. On obtient 1,45 g de N,N-diéthyl (chloro-3 phényl)-1 isoquinoléinecarboxamide-3 fondant à 116°C.

L'acide (chloro-3 phényl)-1 isoquinoléinecarboxylique-3 peut être préparé par action, à la température ambiante, de chlorure d'aluminium (0,369 mole) sur la (chloro-3 phényl)-2 phénylméthylène-4 4H-oxazolone-5 (0,123 mole) dans 500 ml de tétrachloroéthane. Il a un point de fusion de 236°C.

Exemple 12

N,N-diéthyl (méthyl-4 phényl)-1 isoquinoléinecarboxamide-3.

On opère comme dans l'exemple 7 à partir de 5 g d'acide (méthyl-4 phényl)-1 isoquinoléinecarboxylique-3, de 2,18 g de tiréthylamine, de 2,39 g de chloroformiate d'éthyle et de 1,83 g de diéthylamine dans 190 ml de chloroforme. On obtient 3,25 g de N,N-diéthyl (méthyl-4 phényl)-1 isoquinoléinecarboxamide-3 fondant à 113°C.

L'acide (méthyl-4 phényl)-1 isoquinoléinecarboxylique-3 peut être obtenu par action, à 60°C, du chlorure d'aluminium (0,138 mole) sur la (méthyl-4 phényl)-2 phénylméthylène-4 4H-oxazolone-5 (0,046 mole) dans 200 ml de tétrachloroéthane. Il a un point de fusion de 204°C.

Exemple 13

{[(Chloro-2 phényl)-1 isoquinolyl-3] carbonyl }-1 pipéridine.

On opère comme dans l'exemple 7 à partir de 1,42 g d'acide (chloro-2 phényl)-1 isoquinoléinecarboxylique-3, de 0,58 g de triéthylamine, de 0,62 g de chloroformiate d'éthyle et de 0,55 g de pipéridine dans 50 ml de chloroforme. On obtient

1,2 g de {[(chloro-2 phényl)-1 isoquinolyl-3] carbonyl }-1 pipéridine fondant à 161°C.

Exemple 14

N,N-Dipropyl (chloro-2 phényl)-1 isoquinoléinecarboxamide-3.

On opère comme dans l'exemple 7 à partir de 1,42 g d'acide (chloro-2 phényl)-1 isoquinoléinecarboxylique-3, de 0,58 g de triéthylamine, de 0,62 g de chloroformiate d'éthyle et de 0,66 g de N-propyl propylamine dans 50 ml de chloroforme. On obtient 1,40 g de N,N-dipropyl (chloro-2 phényl)-1 isoquinoléinecarboxamide-3 fondant à 101°C.

Exemple 15

N,N-Diéthyl (fluoro-3 phényl)-1 isoquinoléinecarboxamide-3.

On opère comme dans l'exemple 7 à partir de 1,32 g d'acide (fluoro-3 phényl)-1 isoquinoléinecarboxylique-3, de 0,57 g de triéthylamine, de 0,62 g de chloroformiate d'éthyle et de 0,47 g de diéthylamine dans 50 ml de chloroforme. On obtient 1,86 g de N,N-diéthyl (fluoro-3 phényl)-1 isoquinoléinecarboxamide-3 fondant à 92°C.

L'acide (fluoro-3 phényl)-1 isoquinoléinecarboxylique-3 est obtenu par action, à la température ambiante, du chlorure d'aluminium (0,219 mole) sur la (fluoro-3 phényl)-2 phénylméthylène-4 4H-oxazolone-5 (0,073 mole) dans 120 ml de tétrachloroéthane. Il a un point de fusion de 240°C.

Exemple 16

N-Méthyl N-(méthyl-1 propyl) (fluoro-2 phényl)-1 isoquinoléinecarboxamide-3.

On opère comme dans l'exemple 7 à partir de 2,3 g d'acide (fluoro-2 phényl)-1 isoquinoléinecarboxylique-3, de 1 g de triéthylamine, de 1,06 g de chloroformiate d'éthyle et de 0,96 g de N-méthyl butanamine-2 dans 85 ml de chloroforme. On obtient 1,85 g de N-méthyl N-(méthyl-1 propyl) (fluoro-2 phényl)-1 isoquinoléinecarboxamide-3 fondant à 85°C.

L'acide (fluoro-2 phényl)-1 isoquinoléinecarboxylique-3 est obtenu par action, à la température ambiante, de chlorure d'aluminium (0,3 mole) sur la (fluoro-2 phényl)-2 phénylméthylène-4 4H-oxazolone-5 (0,1 mole) dans 220 ml de tétrachloroéthane. Il a un point du fusion de 238°C.

Exemple 17

N-méthyl N-phénylméthyl (chloro-2 phényl)-1 isoquinoléinecarboxamide-3.

On opère comme dans l'exemple 7 à partir de 2,83 g d'acide (chloro-2 phényl)-1 isoquinoléinecarboxylique-3, de 1,15 g de triéthylamine, de 1,24 g de chloroformiate d'éthyle et de 1,56 g de N-méthyl benzylamine dans 100 ml de chloroforme. On obtient 2,5 g de N-méthyl N-phénylméthyl (chloro-2 phényl)-1 isoquinoléinecarboxamide-3 fondant à 128°C.

Exemple 18

N-Cyclohexyl N-méthyl (chloro-2 phényl)-1 isoquinoléinecarboxamide-3.

On opère comme dans l'exemple 7 à partir de 2,83 g d'acide (chloro-2 phényl)-1 isoquinoléinecarboxylique-3, de 1,15 g de triéthylamine, de 1,24 g de chloroformiate d'éthyle et de 1,46 g de N-méthyl cyclohexylamine dans 100 ml de chloroforme. On obtient 2,45 g de N-cyclohexyl N-méthyl (chloro-2 phényl)-1 isoquinoléinecarboxamide-3 fondant à 131°C.

Exemple 19

N-méthyl N-(méthyl-1 propyl) (chloro-2 phényl)-1 méthyl-7 isoquinoléinecarboxamide-3.

On porte à ébullition 3 g d'acide (chloro-2 phényl)-1 méthyl-7 isoquinoléinecarboxylique-3 dans 30 ml de chlorure de thionyle. Après réaction, on évapore le chlorure de thionyle et reprend le résidu dans 30 ml de toluène que l'on évapore de nouveau. On redissout le résidu dans 30 ml de toluène et ajoute une solution de 2,61 g de N-méthyl butanamine-2 dans 10 ml de toluène. Après 4 heures, on évapore le toluène, reprend le résidu dans du chlorure de méthylène, lave la phase organique avec une solution aqueuse de carbonate de sodium, la sèche sur du sulfate de magnésium et l'évapore à sec sous pression réduite. On chromatographie le résidu sur du gel de silice au moyen d'un mélange cyclohexane-acétate d'éthyle (1:1) comme éluant. Après recristallisation dans l'éther, on obtient 1,95 g de N-méthyl N-(méthyl-1 propyl) (chloro-2 phényl)-1 méthyl-7 isoquinoléinecarboxamide-3 fondant à 132°C.

L'acide (chloro-2 phényl)-1 méthyl-7 isoquinoléinecarboxylique-3 est obtenu par action, à la température ambiante, du chlorure d'aluminium (0,219 mole) sur la (chloro-2 phényl)-2 (méthyl-4 phénylméthylène)-4 4H-oxazolone-5 (0,073 mole) dans 160 ml de tétrachloroéthane. Il a un point de fusion de 160°C.

Exemple 20

N,N-diéthyl (chloro-2 phényl)-1 méthyl-7 isoquinoléinecarboxamide-3.

On opère comme à l'exemple 19 à partir de 3 g d'acide (chloro-2 phényl)-1 méthyl-7 isoquinoléinecarboxylique-3, de 30 ml de chlorure de thionyle, de 1,46 g de diéthylamine et de 40 ml de toluène.

On obtient 2,08 g de N,N-diéthyl (chloro-2 phényl)-1 méthyl-7 isoquinoléinecarboxamide-3 fondant à 162°C.

Exemple 21

N-méthyl N-(méthyl-1 propyl) (chloro-2 phényl)-1 isoquinoléinecarboxamide-3.

On opère comme dans l'exemple 7 à partir de 1,68 g d'acide (chloro-2 phényl)-1 isoquinoléinecarboxylique-3, de 0,7 g de triéthylamine, de 0,75 g de chloroformiate d'éthyle et de 0,68 g de N-méthyl butanamine-2 dans 60 ml de chloroforme. On obtient 1,4 g de N-méthyl N-(méthyl-1

propyl) (chloro-2 phényl)-1 isoquinoléinecarboxamide-3 fondant à 136°C.

Exemple 22

N,N-diéthyl chloro-7 (chloro-2 phényl)-1 isoquinoléinecarboxamide-3.

On opère comme dans l'exemple 7 à partir de 3,18 g d'acide chloro-7 (chloro-2 phényl)-1 isoquinoléinecarboxylique-3, de 1,16 g de triéthylamine, de 1,25 g de chloroformiate d'éthyle et de 0,95 g de diéthylamine dans 100 ml de chloroforme. On obtient 2,2 g de N,N-diéthyl chloro-7 (chloro-2 phényl)-1 isoquinoléinecarboxamide-3 fondant à 145°C.

L'acide chloro-7 (chloro-2 phényl)-1 isoquinoléinecarboxylique-3 est obtenu, par action, à 60°C, du chlorure d'aluminium (0,33 mole) sur la (chloro-2 phényl)-2 (chloro-4 phénylméthylène)-4 4H-oxazolone-5 (0,11 mole) dans 460 ml de tétrachloroéthane. Il a un point de fusion de 180–182°C.

Exemple 23

{[(Chloro-2 phényl)-1 isoquinolyl]-3 carbonyl}-1 méthyl-4 piperazine.

On opère comme dans l'exemple 7 à partir de 2,13 g d'acide (chloro-2 phényl)-1 isoquinoléinecarboxylique-3, de 0,88 g de triéthylamine, de 0,95 g de chloroformiate d'éthyle et de 0,95 g de N-méthylpipérazine dans 75 ml de chloroforme. On obtient 1,05 g de {[(chloro-2 phényl)-1 isoquinolyl]-3 carbonyl}-1 méthyl pipérazine fondant à 139°C.

Exemple 24

N,N-Di(méthyl-1 Ethyl) (chloro-2 phényl)-1 isoquinoléinecarboxamide-3.

On opère comme dans l'exemple 7 à partir de 2,83 g d'acide (chloro-2 phényl)-1 isoquinoléinecarboxylique-3, de 1,17 g de triéthylamine, de 1,25 g de chloroformiate d'éthyle et de 1,31 g de diisopropylamine dans 100 ml de chloroforme. On obtient 0,85 g de N,N-di(méthyl-1 éthyl) (chloro-2 phényl)-1 isoquinoléinecarboxamide-3 fondant à 161°C.

Exemple 25

N-méthyl N-(méthyl-1 propen-2 yl) (chloro-2 phényl)-1 isoquinoléinecarboxamide-3.

On opère comme dans l'exemple 7 à partir de 9,2 g d'acide (chloro-2 phényl)-1 isoquinoléinecarboxylique-3, de 3,8 g de triéthylamine, de 4,08 g de chloroformiate d'éthyle et de 3,6 g de N-méthyl méthyl-1 propen-2 ylamine dans 325 ml de chloroforme. On obtient 7,9 g de N-méthyl N(-méthyl-1 propen-2 yl) (chloro-2 phényl)-1 isoquinoléinecarboxamide-3 fondant à 108°C.

Exemple 26

N-méthyl N-(méthyl-1 propyl) (thiényl-2)-1 isoquinoléinecarboxamide-3.

On opère comme dans l'exemple 7 à partir de 3 g d'acide (thiényl-2)-1 isoquinoléinecarboxylique-3, de 1,73 g de triéthylamine, de 1,86 g de chloroformiate d'éthyle et de 1,69 g de N-méthyl butanamine-2 dans 150 ml de chloroforme. On obtient 2,5 g de N-méthyl N-(méthyl-1 propyl) (thiényl-2)-1 isoquinoléinecarboxamide-3 fondant à 146°C.

L'acide (thiényl-2)-1 isoquinoléinecarboxylique-3 est obtenu par action, à la température ambiante, du chlororure d'aluminium (0,42 mole) sur la phénylméthylène-4 (thiényl-2)-2 4H-oxazolone-5 (0,14 mole) dans 300 ml de tétrachloroéthane. Il a un point de fusion de 162°C.

Exemple 27

N,N-diéthyl (chloro-4 phényl)-1 isoquinoléinecarboxamide-3.

On opère comme dans l'exemple 7 à partir de 2,83 g d'acide (chloro-4 phényl)-1 isoquinoléinecarboxylique-3, de 1,15 g de triéthylamine, de 1,24 g de chloroformiate d'éthyle et de 0,95 g de diéthylamine dans 100 ml de chloroforme. On obtient 1,55 g de N,N-diéthyl (chloro-4 phényl)-1 isoquinoléinecarboxamide-3 fondant à 122°C.

L'acide (chloro-4 phényl)-1 isoquinoléinecarboxylique-3 est obtenu à partir de la (chloro-4 phényl)-1 méthyl-3 isoquinoléine. Au moyen de la N-bromosuccinimide, on forme tout d'abord la (chloro-4 phényl)-1 dibromométhyl-3 isoquinoléine que l'on oxyde ensuite avec du nitrate d'argent tout d'abord en milieu neutre, puis en milieu basique. Cet acide a un point de fusion de 232–234°C.

Exemple 28

N,N-diéthyl diméthoxy-6,7 phényl-1 isoquinoléinecarboxamide-3.

On opère comme dans l'exemple 4 à partir de 1,26 g de diméthoxy-6,7, phényl-1 isoquinoléinecarboxylate-3 d'éthyle, de 0,81 g de diéthylamine et de 4,6 ml de butyllithium, 1,6 M dans l'hexane, dans 50 ml de tétrahydrofuranne. On obtient 0,95 g de N,N-diéthyl diméthoxy-6,7 phényl-1 isoquinoléinecarboxamide-3 fondant à 134°C.

Le diméthoxy-6,7 phényl-1 isoquinoléinecarboxylate-3 d'éthyle peut être préparé selon T. Hosono, J. Pharm. Soc. Japan 65, N° 7/8A, 11, (1945).

Exemple 29

N-méthyl N-(méthyl-1 propyl) phényl-7 thièno [2,3-c] pyridinecarboxamide-5.

On opère comme dans l'exemple 7 à partir de 3,82 g d'acide phényl-7 thièno [2,3-c] pyridinecarboxylique-5, de 1,74 g de triéthylamine, de 1,87 g de chloroformiate d'éthyle et de 1,7 g de N-méthyl butanamine-2 dans 150 ml de chloroforme. On obtient 3,15 g de N-méthyl N-(méthyl-1 propyl) phényl-7 thièno [2,3-c] pyridinecarboxamide-5 fondant à 117°C.

L'acide phényl-7 thièno [2,3-c] pyridinecarboxylique-5 est obtenu par action, à la température ambiante, du chlorure d'aluminium (0,225 mole) sur la phényl-2 [thiényl-3 méthylène]-4 4H-oxazolone-5 (0,075 mole) dans 150 ml de tétrachloroéthane. Il a un point de fusion de 208°C.

Exemple 30

N-méthyl N-(méthyl-1 propyl) phényl-4 thièno [3,2-c] pyridinecarboxamide-6.

On opère comme dans l'exemple 7 à partir de de 3,82 g d'acide phényl-4 thièno [3,2-c] pyridinecarboxylique-6, de 1,74 g triéthylamine, de 1,87 g de chloroformiate d'éthyle et de 1,7 g de N-méthyl butanamine-2 dans 150 ml de chloroforme. On obtient 1,9 g de N-méthyl N-(méthyl-1 propyl) phényl-4 thièno [3,2-c] pyridinecarboxamide-6 fondant à 104°C.

L'acide phényl-4 thièno [3,2-c] pyridinecarboxylique-6 est obtenu par action, à la température ambiante, du chlorure d'aluminium (0,352 mole) sur la phényl-2 [thiènyl-2 méthylène]-4 4H-oxazolone-5 (0,117 mole) dans 235 ml de tétrachloroéthane. Il a un point de fusion de 165°C.

Exemple 31

N,N-diéthyl (chloro-2 phényl)-4 thièno [3,2-c] pyridinecarboxamide-6.

On opère comme dans l'exemple 7 à partir de 2,9 g d'acide (chloro-2 phényl)-4 thièno [3,2-c] pyridinecarboxylique-6, de 1,17 g de triéthylamine, de 1,25 g de chloroformiate d'éthyle et de 0,94 g de diéthylamine dans 100 ml de chloroforme. On obtient 2,1 g de N,N-diéthyl (chloro-2 phényl)-4 thièno [3,2-c] pyridinecarboxamide-6 fondant à 119°C.

L'acide (chloro-2 phényl)-4 thièno [3,2-c] pyridinecarboxylique-6 est obtenu par action, à 60 °C, du chlorure d'aluminium (0,103 mole) sur la (chloro-2 phényl)-2 [thiènyl-2 méthylène]-4 4H oxazolone-5 (0,034 mole) dans 145 ml de tétrachloro-1,1,2,2 ethane. Il a un point de fusion de 210°C.

Exemple 32

N,N-diéthyl (chloro-2 phényl)-7 thièno [2,3-c] pyridinecarboxamide-5.

On opère comme dans l'exemple 7 à partir de 2,9 g d'acide (chloro-2 phényl)-7 thièno [2,3-c] pyridinecarboxylique-5, de 1,17 g de triéthylamine, de 1,25 g de chloroformiate d'éthyle et de 0,94 g de diéthylamine dans 100 ml de chloroforme. On obtient 1,4 g de N,N-diéthyl (chloro-2 phényl)-7 thièno [2,3-c] pyridinecarboxamide-5 fondant à 86°C.

L'acide (chloro-2 phényl)-7 thièno [2,3-c] pyridinecarboxylique-5 est obtenu par action, à 60°C, du chlorure d'aluminium (0,342 mole) sur la (chloro-2 phényl)-2 [thiènyl-3 méthylène]-4 4H oxazolone-5 (0,114 mole) dans 475 ml de tétrachloro-1,1,2,2 éthane. Il a un point de fusion de 152°C.

Exemple 33

N,N-diéthyl phényl-4 quinazolinecarboxamide-2.

On opère comme dans l'exemple 19 à partir de 2,5 g d'acide phényl-4 quinazolinecarboxylique-2, de 10 ml de chlorure de thionyle, de 25 ml de toluène et de 25 ml de diéthylamine. On obtient 1,27 g de N,N-diéthyl phényl-4 quinazolinecarboxamide-2 fondant à 101°C.

L'acide phényl-4 quinazolinecarboxylique-2 est obtenu par action, du chlorure de méthyloxalyle sur l'amino-2 benzophénone dans la pyridine. La N(méthyloxalyl) benzoyl-2 aniline ainsi formée étant ensuite traitée par l'acétate d'ammonium dans l'acide acétique à l'ébullition. L'acide a un point de fusion du 167°C.

Exemple 34

N-méthyl N-(méthyl-1 propyl) phényl-4 quinazolinecarboxamide-2.

On opère comme dans l'exemple 19 à partir de 2,5 g d'acide phényl quinazolinecarboxylique-2, de 10 ml de chlorure de thionyle, de 25 ml de toluène et de 10 ml de N-méthyl butanamine-2. On obtient 2,2 g de N-méthyl N-(méthyl-1 propyl) phényl-4 quinazolinecarboxamide-2 fondant à 128°C.

Exemple 35

N-méthyl N-(méthyl-1 propyl) (fluoro-2 phényl)-4 quinoléinecarboxamide-2.

On porte à ébullition 3,3 g d'acide (fluoro-2 phényl)-4 quinoléinecarboxylique-2 dans 10 ml de chlorure de thionyle.

Après reaction, on évapore le chlorure de thionyle et reprend le résidu dans 30 ml de toluène que l'on évapore de nouveau.

Le résidu est mis en suspension dans 100 ml de toluène. On ajoute alors 4,8 ml de triéthylamine, puis sous agitation, 1,8 g de chlorhydrate de N-méthyl butanamine-2. On agite 14 heures à température ambiante.

Le milieu réactionnel est repris par 100 ml d'eau. La phase toluènique est décantée: la phase aqueuse est extraite par 3 fois 100 ml d'éther. Les phases organiques sont rassemblées, lavées par 2 fois 50 ml d'eau, séchées sur sulfate de magnésium et concentrées sous pression réduite. Le résidu est chromatographié sur du gel de silice au moyen d'un mélange cyclohexane/acétate d'éthyle 70 : 30. Après cristallisation de l'huile obtenue dans un mélange éther isopropylique-éther 5 : 2, on obtient 1,16 g de N-méthyl N(méthyl-1 propyl) (fluoro-2 phényl)-4 quinoléinecarboxamide-2 fondant à 86°C.

L'acide (fluoro-2 phényl)-4 quinoléinecarboxylique-2 est obtenu par action de l'acide chlorhydrique concentré (400 ml) sur la tribromométhyl-2 (fluoro-2 phényl)-4 quinoléine pendant 48 heures au reflux. Il présente un point de fusion égal à 210°C.

La tribromométhyl-2 (fluoro-2 phényl)-4 quinoléine est obtenue par action du brome ($2,7\ 10^{-1}$ mole) en solution dans l'acide acétique (36 ml) sur la (fluoro-2 phényl)-4 méthyl-2 quinoléine ($4,6\ 10^{-2}$ mole) en solution dans 55 ml d'acide acétique en présence d'acétate de sodium ($1,8\ 10^{-1}$ mole) et d'anhydride acétique ($4,9\ 10^{-2}$ mole).

La (fluoro-2 phényl)-4 méthyl-2 quinoléine peut être obtenue par action de l'acétone ($5,6\ 10^{-2}$ sur l'(amino-2 phényl) (fluoro-2 phényl) méthanone ($5,6\ 10^{-2}$ mole) dans l'acide acétique (60 ml), en présence d'acide sulfurique (0,6 ml). Elle présente un point de fusion égal à 119°C.

Exemple 36

N-méthyl N-(méthyl-1 propyl) (méthoxy-4 phényl)-4 quinoléinecarboxamide-2.

On opère comme dans l'exemple 35 en partant de 2,1 g d'acide (méthoxy-4 phényl)-4 quinoléinecarboxylique-2, de 6,4 ml de chlorure de thionyle et de 1,4 g de chlorhydrate de N-méthyl butanamine-2.

Après chromatographie sur gel de silice au moyen d'un mélange cyclohexane-acétate d'éthyle (60 : 40) comme éluant et recristallisation dans l'éther isopropylique, on obtient 0,77 g de N-méthyl N-(méthyl-1 propyl) (méthoxy-4 phényl)-4 quinoléinecarboxamide-2 fondant à 114°C.

L'acide (méthoxy-4 phényl)-4 quinoléinecarboxylique-2 peut être obtenu par une double oxydation de la (méthoxy-4 phényl)-4 méthyl-2 quinoléine (5,6 $10^{-2}$ mole) tout d'abord en (méthoxy-4 phényl)-4 quinoléinecarboxaldéhyde-2 au moyen du dioxyde de sélénium (5,6 $10^{-2}$ mole) dans 50 ml d'un mélange dioxanne-eau 9 : 1, puis en acide (méthoxy-4 phényl)-4 quinoléinecarboxylique-2 par action sur l'aldéhyde brute obtenue précédemment (2,2 $10^{-2}$ mole) de l'eau oxygénée à 110 vol. (18ml) au sein de l'acétone (200 ml).

L'acide (méthoxy-4 phényl)-4 quinoléinecarboxilique-2 présente un point de fusion égal à 213°C; on observe néanmoins une première fusion puis ressolidification à partir de 130°C.

La (méthoxy-4 phényl)-4 méthyl-2 quinoléine peut être préparée par action de l'acétone (1,48 $10^{-1}$ mole) sur l'(amino-2 phényl) (méthoxy-4 phényl) méthanone (7,4 $10^{-2}$ mole) dans l'acide acétique (85 ml), en présence d'acide sulfurique (0,85 ml). Elle a un point de fusion de 101°C. L'(amino-2 phényl) (méthoxy-4 phényl) méthanone peut être préparée selon Ullmann et Bleier Ber, 35, 4278, (1902).

Exemple 37

N,N-diéthyl (méthoxy-4 phényl)-4 quinoléinecarboxamide-2.

On procède comme à l'exemple 1, en partant de 21 g d'acide (méthoxy-4 phényl)-4 quinoléinecarboxylique-2, de 64 ml de chlorure de thionyle et de 39 ml de diéthylamine. On isole après traitement 2,2 g de N,N-diéthyl (méthoxy-4 phényl)-4 quinoléinecarboxamide-2.

Après recristallisation dans l'éther isopropylique, le produit présente un point de fusion égal à 91°C.

Exemple 38

N,N-diéthyl (nitro-3 phényl)-4 quinoléinecarboxamide-2.

On procède comme à l'exemple 1 en partant de 57 g d'acide (nitro-3 phényl)-4 quinoléinecarboxylique-2, de 28,5 ml de chlorure de thionyle et de 9,9 ml de diéthylamine dans 50 ml de toluène.

Après une première chromatographie sur gel de silice au moyen d'un mélange chloroforme-éther (90 : 10) comme éluant, puis une seconde chromatographie sur la même support, mais en utilisant un mélange cyclohexane-diéthylamine (90 : 10), on obtient 1 g de N,N-diéthyl (nitro-3 phényl)-4 quinoléinecarboxamide-2 sous forme d'une laque brune.

Spectre R.M.N. du produit obtenu:

protons des noyaux aromatiques: δ 7,3 à 8,4 ppm,

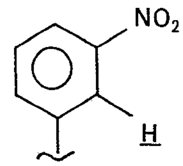

δ = 8,4 ppm

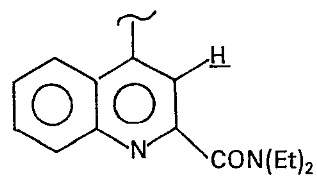

δ = 7,6 ppm

L'acide (nitro-3 phényl)-4 quinoléinecarboxylique-2 peut être préparé par analogie avec l'acide (méthoxy-4 phényl)-4 quinoléinecarboxylique-2 décrit à l'exemple 36 mais en partant de méthyl-2 (nitro-3 phényl)-4 quinoléine (2,46 $10^{-2}$ mole), de 2,73 g de dioxyde de sélénium et de 20,9 ml d'eau oxygénée à 110 vol.

La méthyl-2 (nitro-3 phényl)-4 quinoléine est préparée par nitration à 0° de la méthyl-2 phényl-4 quinoléine (1,36 $10^{-1}$ mole) au moyen d'un mélange sulfonitrique composé de 90 ml d'acide nitrique à 60% et de 90 ml d'acide sulfurique concentré, puis séparation des isomères obtenues. Elle fond à 157°C.

La méthyl-2 phényl-4 quinoléine peut être préparée selon Geigy et Koenigs Ber, 18, 2406, (1885).

Exemple 39

N,N-dibutyl phényl-1 isoquinoléinecarboxamide-3.

On opère comme dans l'exemple 7 à partir de 2,5 g d'acide phényl-1 isoquinoléinecarboxylique-3, de 1,15 g de triéthylamine, de 1,24 g de chloroformiate d'éthyle et de 1,66 g dibutylamine dans 100 ml de toluène. Après chromatographie et recristallisation dans l'éther de pétrole, on obtient 1,5 g de N,N-dibutyl phényl-1 isoquinoléinecarboxamide-3 fondant à 62°C.

Exemple 40

Ethyl-2 [(phényl-1 isoquinolyl-3) carbonyl]-1 pipéridine.

On opère comme dans l'exemple 7 à partir de 2,5 g d'acide phényl-1 isoquinoléinecarboxylique-3, de 1,15 g de triéthylamine, de 1,24 g de chloroformiate d'éthyle et de 1,46 g d'éthyl-2 pipéridine dans 100 ml de toluène. Après recristallisation dans l'éther de pétrole, on obtient 2,2 g d'éthyl-2 [(phényl-1 isoquinolyl-3) carbonyl]-1 pipéridine fondant à 86°C.

Exemple 41

Ethyl-2 (R) [(phényl-1 isoquinolyl-3) carbonyl]-1 pipéridine.

On opère comme dans l'exemple 7 à partir de 2,5 g d'acide phényl-1 isoquinoléinecarboxylique-3, de 2,86 g de tiréthylamine, de 1,24 g de chloroformiate d'éthyle et de 1,93 g de chlorhydrate d'éthyl-2 (R) pipéridine dans 100 ml de toluène. On obtient, après chromatographie, 2 g d'éthyl-2 (R) [(phényl-1 isoquinolyl-3) carbonyl]-1 pipéridine sous forme d'huile $[\alpha]_D^{26} = +1{,}4°$ (2% dans l'éthanol).

Exemple 42

Ethyl-2 (S) [(phényl-1 isoquinolyl-3) carbonyl]-1 pipéridine.

On opère comme dans l'exemple 7 à partir de 2,5 g d'acide phényl-1 isoquinoléinecarboxylique-3 de 1,15 g de triéthylamine, de 1,24 g de chloroformiate d'éthyle et de 1,2 g d'éthyl-2 (S) pipéridine dans 100 ml de toluène. On obtient, après chromatographie, 3 g d'éthyl-2 (S) [(phényl-1 isoquinolyl-3) carbonyl]-1 pipéridine sous forme d'huile. $[\alpha]_D^{23} = -2{,}4°$ (2% dans l'éthanol).

Exemple 43

N,N-diéthyl (trifluorométhyl-3 phényl)-1 isoquinoléinecarboxamide-3.

On opère comme dans l'exemple 7 à partir de 1,55 g d'acide (trifluorométhyl-3 phényl)-1 isoquinoléinecarboxylique-3, de 0,57 g de triéthylamine, de 0,62 g de chloroformiate d'éthyle et de 0,48 g de diéthylamine dans 50 ml de toluène. On obtient, après recristallisation dans l'éther de pétrole, 0,95 g de N,N-diéthyl (trifluorométhyl-3 phényl)-1 isoquinoléinecarboxamide-3 fondant à 76°C.

L'acide (trifluorométhyl-3 phényl)-1 isoquinoléinecarboxylique-3 peut être obtenu à partir de la méthyl-3 (trifluorométhyl-3 phényl)-1 isoquinoléine. Au moyen de la N-bromosuccinimide, on forme la dibromométhyl-3 (trifluorométhyl-3 phényl)-1 isoquinoléine que l'on traite par du nitrate d'argent d'abord en milieu neutre, puis en milieu alcalin. Cet acide a un point de fusion de 170°C.

Exemple 44

N-méthyl N[méthyl-1 (S) propyl] (chloro-2 phényl)-1 isoquinoléinecarboxamide-3.

On opère comme dans l'exemple 7 à partir de 1,7 g d'acide (chloro-2 phényl)-1 isoquinoléinecarboxylique-3, de 2,1 g de triéthylamine, de 0,75 g de chloroformiate d'éthyle et de 0,96 g de chlorhydrate de N-méthyl butanamine-2 (S) dans 60 ml de toluène. Après chromatographie et recristallisation dans le cyclohexane, on obtient 0,5 g de N-méthyl N-[méthyl-1 (S) propyl] (chloro-2 phényl)-1 isoquinoléinecarboxamide-3 fondant à 134°C. $[\alpha]_D^{27} = +53{,}7°$ (0,5% dans le chloroforme).

Exemple 45

N-méthyl N-[méthyl-1 (R) propyl] (chloro-2 phényl)-1 isoquinoléinecarboxamide-3.

On opère comme dans l'exemple 7 à partir de 1,7 g d'acide (chloro-2 phényl)-1 isoquinoléinecarboxylique-3, de 0,75 g de chloroformiate d'éthyle de 2,1 g de triéthylamine et de 0,96 g de chlorhydrate de N-méthylbutanamine-2 (R) dans 60 ml de toluène. Après chromatographie et recristallisation dans le cyclohexane, on obtient 0,6 g de N-méthyl N-[méthyl-1 (R) propyl] (chloro-2 phényl)-1 isoquinoléinecarboxamide-3 fondant à 134°C. $[\alpha]_D^{27} = -51°$ (0,5% dans le chloroforme).

Exemple 46

N-méthyl N-(méthyl-1 propyl) (chloro-2 phényl)-7 thièno [2,3-c] pyridinecarboxamide-5.

On opère comme dans l'exemple 7 à partir de 3,5 g d'acide (chloro-2 phényl)-7 thièno [2,3-c] pyridinecarboxylique-5 de 5,85 ml de triéthylamine, de 1,33 ml de chloroformiate d'éthyle et de 1,94 g de chlorhydrate de N-méthyl butanamine-2 dans 60 ml de chloroforme. On obtient après recristallisation dans l'éther de pétrole, 0,6 g de N-méthyl N(méthyl-1 propyl) (chloro-2 phényl)-7 thièno [2,3-c] pyridinecarboxamide-5 fondant à 97°C.

Exemple 47

N-méthyl N-(méthyl-1 propyl) (chloro-2 phényl)-4 thièno [3,2-c] pyridinecarboxamide-6.

On opère comme dans l'exemple 7 à partir de 10 g d'acide (chloro-2 phényl)-4 thièno [3,2-c] pyridinecarboxylique-6, de 11,9 ml de triéthylamine, de 4 ml de chloroformiate d'éthyle et de 4,8 ml de N-méthyl butanamine-2 dans 200 ml de chloroforme.

On obtient, après chromatographie et cristallisations dans l'éther de pétrole, 1 g de N-méthyl N-(méthyl-1 propyl) (chloro-2 phényl)-4 thièno [3,2-c] pyridinecarboxamide-6 fondant à 82°C.

Propriétés Pharmacologiques

Affinité pour les sites récepteurs «périphériques» des benzodiazépines.

Cette affinité est mesurée par l'aptitude des produits à déplacer le diazépam tritié ($^3$H-diazépam) de son site de liaison et est exprimée par une valeur Ki, en nanomoles (nM) qui est calculée par la formule:

$$Ki = \frac{IC_{50}}{1 + \frac{C}{KD}}$$

dans laquelle C représente la concentration de $^3$H-diazépam utilisée (1mM), $K_D$ une constante d'affinité égale à 15 nM et $IC_{50}$ la concentration nécessaire pour obtenir une inhibition de 50% la liaison du $^3$H-diazépam.

Les produits ont été étudiés selon le protocole de C. Braestrup et Coll., Proc. Natl, Acad. Sci, USA, 1977, 74, 3805, sur des membranes de rein de rat.

A titre d'exemple, on a obtenu les résultats suivants:

| Produits | Ki (nM) |
|---|---|
| Exemple 1 | 5,4 |
| Exemple 3 | 27 |
| Exemple 5 | 150 |
| Exemple 6 | 17 |
| Exemple 7 | 210 |
| Exemple 10 | 2 |
| Exemple 11 | 9 |
| Exemple 12 | 99 |
| Exemple 13 | 72 |
| Exemple 14 | 18 |
| Exemple 15 | 20 |
| Exemple 16 | 0,9 |
| Exemple 17 | 117 |
| Exemple 21 | 8 |
| Exemple 24 | 20 |
| Exemple 25 | 3 |
| Exemple 31 | 9 |
| Exemple 32 | 6,3 |
| Exemple 33 | 27 |
| Exemple 35 | 2 |
| Exemple 38 | 30 |
| Exemple 39 | 21 |
| Exemple 40 | 16 |
| Exemple 41 | 22 |
| Exemple 42 | 13 |
| Exemple 43 | 13 |
| Exemple 44 | 19 |
| Exemple 45 | 9 |
| Exemple 46 | 7 |
| Exemple 47 | 1 |

Propriétés toxicologiques

Les toxicités aiguës des composés selon l'invention ont été déterminées chez la souris mâle CDI (Charles River) par voie orale. Les $DL_{50}$ ont été calculées, après 3 jours d'observation, par la méthode cumulative de J.J. Reed et H. Muench (Amer. J. Hyg. 1938, 27, 493).

Les composés se comportent come des substances relativement peu toxiques chez la souris, puisque les $DL_{50}$ des composés se situent entre 200 et 1,000 mg/kg.

Utilisation thérapeutique

Les composés de l'invention peuvent être utilisés en thérapeutique humaine, sous forme de comprimés, capsules, gélules, suppositoires, solutions ingérables ou injectables, etc.... pour le traitement des troubles pulmonaires, rénaux, cardiovasculaires et circulatoires, pour le traitement des états pathologiques associés à des désordres immunitaires, pour le traitement des états d'aggressivité, ainsi que dans les diverses indications des benzodiazépines (par exemple hypnotiques, anticonvulsivants et anxiolytiques).

La posologie dépend des effets recherchés et de la voie d'administration utilisée. Par exemple, par voie orale, elle peut être comprise entre 20 et 1,000 mg de substance active par jour, avec des doses unitaires allant de 5 à 200 mg.

**Revendications**

1. Les composés de formule générale:

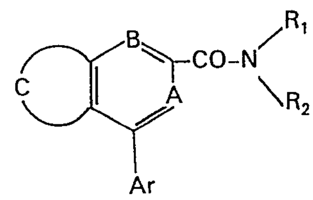

(I)

dans laquelle $R_1$ et $R_2$ représentent indépendamment un groupe alkyle comprenant 1 à 6 atomes de carbone, linéaire ou ramifié, un groupe cycloalkyle comprenant 3 à 7 atomes de carbone, un groupe phénylalkyle ou cycloalkylalkyle, dont la partie alkyle comprend 1 à 3 atomes de carbone; $R_1$ et $R_2$ peuvent représenter également un groupe alcényle ou alcynyle comprenant 3 à 6 atomes de carbone, à condition que la double ou la triple liaison ne soit pas située en position 1-2 par rapprt à l'atome d'azote; $R_1$ et $R_2$ peuvent représenter encore un groupe de formule $-R_3-Z-R_4$ dans laquelle $R_3$ représente un groupe alkylène linéaire ou ramifié de 2 à 6 atomes de carbone, à condition qu'au moins 2 atomes de carbone séparent l'atome d'azote du groupe Z; $R_4$ représente un groupe alkyle comportant de 1 à 4 atomes de carbone et Z l'atome d'oxygène, de soufre ou le groupe $>N-R_5$, $R_5$ représentant l'atome d'hydrogène ou un groupe alkyle de 1 à 3 atomes de carbone; $R_1$ et $R_2$ peuvent former avec l'atome d'azote auquel ils sont rattachés un hétérocycle comprenant éventuellement un second hétéroatome. Ar représente un groupe phényle, pyridyle ou thiènyle ou un groupe phényle substitué par un ou deux substituants pris parmi les atomes d'halogène, les groupes alkyle, alcoxy et alkylthio ayant 1 à 4 atomes de carbone, le groupe trifluorométhyle et le groupe nitro; A et B représentent indépendamment N ou CH-

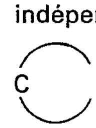

représente l'enchaînement

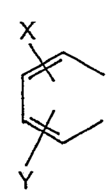

ou, lorsque A représente N et B représente CH, l'un des enchaînements suivants:

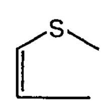

,

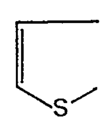

X, Y représentant indépendamment l'atome d'hydrogène, un atome d'halogène, un groupe alkyle ou alkoxy comprenant 1 à 3 atomes de carbone, le groupe nitro ou trifluorométhyle.

2. Composés tels que définis selon la revendication 1 de formule générale:

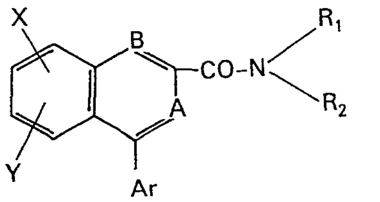
(II)

dans laquelle A, B, Ar, $R_1$, $R_2$, X et Y ont les mêmes significations que dans la formule (I).

3. Composés tels que définis selon la revendication 1 de formule générale:

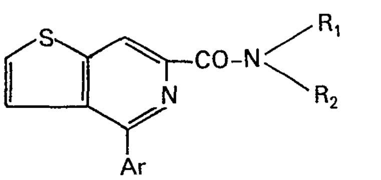
(III)

dans laquelle Ar, $R_1$, $R_2$ ont les mêmes significations que dans la formule (I).

4. Composés tels que définis selon la revendication 1 de formule générale:

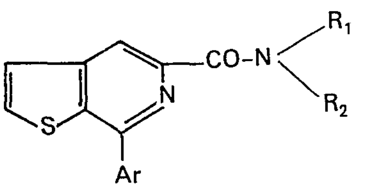
(IV)

dans laquelle Ar, $R_1$, $R_2$ ont les mêmes significations que dans la formule (I).

5. Composés tels que définis selon chacune des revendications 1, 2, 3, 4 dans lesquels

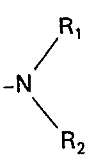

représente un cycle pyrrolidine, pipéridine, morpholine ou celui de la pipérazine, éventuellement substitués sur l'azote par un groupe alkyle comprenant 1 à 3 atomes de carbone.

6. Les énantiomères et les composés racémiques des composés définis sous chacune des revendications 1 à 5.

7. Procédé de préparation des composés de la revendication 1, caractérisé en ce que l'on traite un composé de formule (VI):

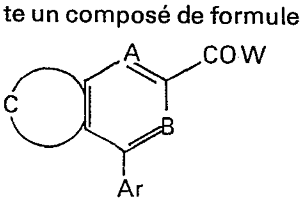
(VI)

avec une amine de formule $T_1$-NH-$R_2$ dans lesquelles A, B, C, Ar, $R_1$ et $R_2$ ont la même signification que ci-dessus et W représente un groupe alcoxy, un atome de chlore ou un groupe alcoxycarbonyloxy.

8. Composés pharmacologiquement actifs possédant de l'affinité pour les sites récepteurs du $^3$H-diazépam, caractérisés en ce qu'ils sont constitués par les composés définis sous chacune des revendications 1 à 6.

9. Médicaments utilisables en particulier pour le traitement des troubles pulmonaires, rénaux, cardiovasculaires ou circulatoires, pour le traitement des états pathologiques associés à des désordres immunitaires, pour le traitement des états d'agressivité, ainsi que dans les applications des benzodiazépines, caractérisés en ce qu'ils comprennent des composés tels que définis sous chacune des revendications 1 à 6.

## Patentansprüche

1. Verbindungen der allgemeinen Formel

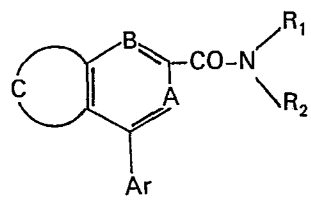
(I)

worin $R_1$ und $R_2$ eine lineare oder verzweigte Alkylgruppe mit 1 bis 6 Kohlenstoffatomen, eine Cycloalkylgruppe mit 3 bis 7 Kohlenstoffatomen, eine Phenylalkyl- oder Cycloalkylalkylgruppe, deren Alkylteil 1 bis 3 Kohlenstoffatome hat, bedeuten; $R_1$ und $R_2$ können auch eine Alkenyl- oder Alkinylgruppe mit 3 bis 6 Kohlenstoffatomen bedeuten, mit der Massgabe, dass die Doppel- oder Dreifachbindung nicht in 1-2-Stellung in Bezug auf das Stickstoffatom steht; $R_1$ und $R_2$ können auch eine Gruppe der Formel $-R_3$-Z-$R_4$ bedeuten, worin $R_3$ eine lineare oder verzweigte Alkylengruppe mit 2 bis 6 Kohlenstoffatomen bedeutet, mit der Massgabe, dass mindestens 2 Kohlenstoffatome das Stickstoffatom von der Gruppe Z trennen; $R_4$ bedeutet eine Alkylgruppe mit 1 bis 4 Kohlenstoffatomen, und Z ein Sauerstoff- oder Schwefelatom oder die Gruppe >N-$R_5$, wobei $R_5$ ein Wasserstoffatom oder eine Alkylgruppe mit 1 bis 3 Kohlenstoffatomen darstellt; $R_1$ und $R_2$ können mit dem Stickstoffatom, an das sie gebunden sind, einen Heterocyclus bedeuten, der gegebenenfalls ein zweites Heteroatom enthält, Ar bedeutet eine Phenyl-, Pyridyl- oder Thienylgruppe oder eine Phenylgruppe, die durch ein oder zwei Substituenten ausgewählt aus den Halogenatomen, Alkyl-, Alkoxy- und Alkylthiogruppen mit 1 bis 4 Kohlenstoffatomen, der Trifluormethylgruppe und der Nitrogruppe substituiert ist; A und B bedeuten unabhängig N oder CH-,

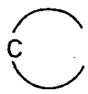

bedeutet die Verkettung

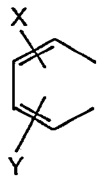

oder, wenn A für N steht und B für CH steht, eine der folgenden Verkettungen:

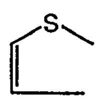 , 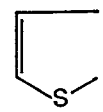

wobei X, Y unabhängig das Wasserstoffatom, ein Halogenatom, eine Alkyl- oder Alkoxygruppe mit 1 bis 3 Kohlenstoffatomen, die Nitro- oder Trifluormethylgruppe bedeuten.

2. Verbindungen, wie in Anspruch 1 definiert, der allgemeinen Formel

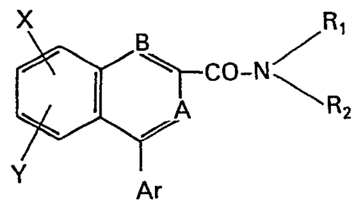

(II)

worin A, B, Ar, $R_1$, X und Y dieselben Bedeutungen wie in Formel (I) haben.

3. Verbindungen, wie in Anspruch 1 definiert der allgemeinen Formel

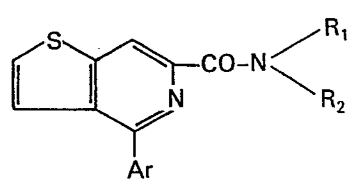

(III)

worin Ar, $R_1$, $R_2$ dieselben Bedeutungen wie in Formel (I) haben.

4. Verbindungen, wie in Anspruch 1 definiert, der allgemeinen Formel

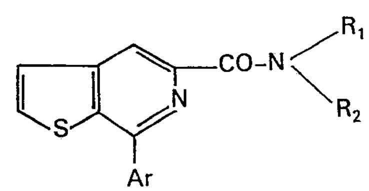

(IV)

worin Ar, $R_1$ und $R_2$ dieselben Bedeutungen wie in Formel (I) haben.

5. Verbindungen, wie in jedem der Ansprüche 1, 2, 3, 4 definiert, worin

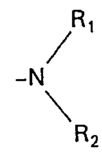

einen Pyrrolidin-, Piperidin-, Morpholinring oder denjenigen des Piperazins gegebenenfalls am Stickstoff durch eine Alkylgruppe 1 bis 3 Kohlenstoffatomen substuiert, bedeutet.

6. Die Enantiomeren und die racemischen Verbindungen der Verbindungen, wie sie in jedem der Ansprüche 1 bis 5 definiert sind.

7. Verfahren zur Herstellung der Verbindungen gemäss Anspruch 1, dadurch gekennzeichnet, dass man eine Verbindung der Formel (VI)

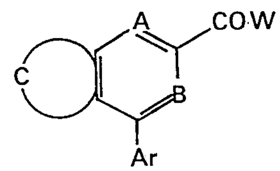

(VI)

mit einem Amin der Formel $R_1$-NH-$R_2$, worin A, B, C, Ar, $R_1$ und $R_2$ dieselbe Bedeutung wie oben haben und W eine Alkoxygruppe, ein Chloratom oder eine Alkoxycarbonyloxygruppe bedeutet, behandelt.

8. Pharmakologisch wirksame Verbindungen mit einer Affinität für die Rezeptorstellen des $^3$H-Diazepams, dadurch gekennzeichnet, dass sie aus den Verbindungen, welche in jedem der Ansprüche 1 bis 6 definiert sind, bestehen.

9. Arzneimittel, verwendbar insbesondere zur Behandlung von Lungen-, Nieren-, cardiovasculären oder Kreislaufstörungen, zur Behandlung von pathologischen Zuständen verbunden mit immunitärer Unordnung, zur Behandlung von Zuständen von Aggressivität sowie bei den Anwendungen der Benzodiazepine, dadurch gekennzeichnet, dass sie Verbindungen, wie sie in jedem der Ansprüche 1 bis 6 definiert sind, umfassen.

## Claims

1. The compounds of general formula:

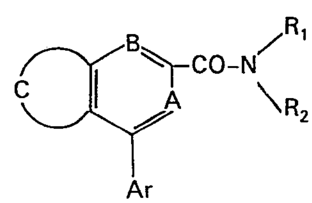

(I)

in which $R_1$ and $R_2$ indenpendently denote a linear or branched alkyl group containing 1 to 6 carbon atoms, a cycloalkyl group containing 3 to 7 carbon atoms, a phenylalkyl or cycloalkylalkyl group in which the alkyl portion contains 1 to 3 carbon atoms; $R_1$ and $R_2$ can also denote an alkenyl or alkynyl group containing 3 to 6 carbon atoms, provided that the double or triple bond is not situated in the 1-2 position relative to the nitrogen at-

om; $R_1$ and $R_2$, can also denote a group of formula $-R_3$-Z-$R_4$ in which $R_3$ denotes a linear or branched alkylene group having 2 to 6 carbon atoms, provided that at least 2 carbon atoms separate the nitrogen atom from the Z group; $R_4$ denotes an alkyl group containing from 1 to 4 carbon atoms and Z an oxygen or sulphur atom or a group >N-$R_5$, $R_5$ denoting a hydrogen atom or an alkyl group having 1 to 3 carbon atoms; $R_1$ and $R_2$ can form with the nitrogen atom to which they are attached a heterocyclic system optionally containing a second hetero atom; Ar denotes a phenyl, pyridyl or thienyl group, or a phenyl group substituted with one or more substituents chosen from halogen atoms, alkyl, alkoxy and alkylthio groups having 1 to 4 carbon atoms, a trifluoromethyl group and a nitro group; A and B independently denote N or CH-;

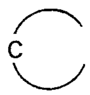

denotes the arrangement

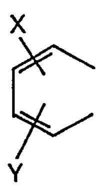

or, when A denotes N and B denotes CH, one of the following arrangements:

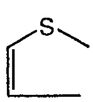 , 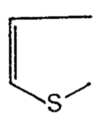

X and Y independently denoting a hydrogen atom, a halogen atom, an alkyl or alkoxy group containing 1 to 3 carbon atoms, or a nitro or trifluoromethyl group.

2. Compounds as defined according to Claim 1 of general formula:

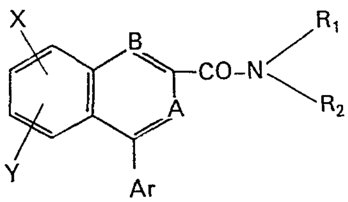

(II)

in which A, B, Ar, $R_1$, $R_2$, X and Y have the same significances as in formula (I).

3. Compounds as defined according to Claim 1 of general formula:

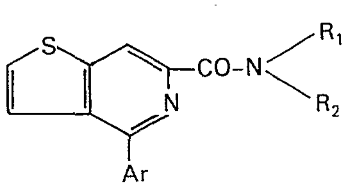

(III)

in which Ar, $R_1$ and $R_2$ have the same significances as in formula (I).

4. Compounds as defined according to Claim 1 of general formula:

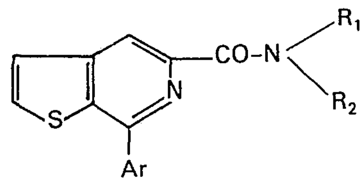

(IV)

in which Ar, $R_1$ and $R_2$ have the same significances as in formula (I).

5. Compounds as defined according to each one of Claimes 1, 2, 3 and 4, in which

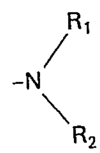

denotes a pyrrolidine, piperidine or morpholine ring, or that of piperazine, optionally substituted on the nitrogen with an alkyl group containing 1 to 3 carbon atoms.

6. The enantiomers and racemic compounds of the compounds defined under each one of Claims 1 to 5.

7. Process for preparing compounds of Claim 1, characterised in that a compound of formula (VI):

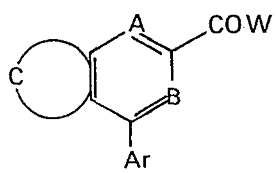

(VI)

is treated with an amine of formula $R_1$-NH-$R_2$, in which A, B, C, Ar, $R_1$ and $R_2$ have the same significance as above and W denotes an alkoxy group, a chlorine atom or alkoxycarbonyloxy group.

8. Pharmacologically active compounds possessing affinity for the [$^3$H]-diazepam receptor sites, characterised in that they consist of the compounds defined under each one of Claims 1 to 6.

9. Drugs which can be used, in particular, for treating pulmonary, renal, cardiovascular or circulatory disorders, for treating the pathological states associated with immunity disorders and for treating aggressive states, as well as in the applications of benzodiazepines, characterised in that they contain compounds such as defined under each one of Claims 1 to 6.